(19) Europäisches Patentamt
European Patent Office
Office européen des brevets

(11) Veröffentlichungsnummer: **0 097 844**
**A1**

# (12) EUROPÄISCHE PATENTANMELDUNG

(21) Anmeldenummer: **83105538.9**

(22) Anmeldetag: **06.06.83**

(51) Int. Cl.³: **C 07 C 69/612**
C 07 C 69/734, C 07 C 69/743
A 01 N 31/14, A 01 N 53/00

(30) Priorität: **18.06.82 DE 3222910**

(43) Veröffentlichungstag der Anmeldung:
**11.01.84 Patentblatt 84/2**

(84) Benannte Vertragsstaaten:
**CH DE FR GB LI**

(71) Anmelder: **BAYER AG**
**Zentralbereich Patente, Marken und Lizenzen**
**D-5090 Leverkusen 1, Bayerwerk(DE)**

(72) Erfinder: **Führer, Wolfgang, Dr.**
**Wehrstrasse 28**
**D-5202 Hennef 1(DE)**

(72) Erfinder: **Stetter, Jörg, Dr.**
**Gellertweg 4**
**D-5600 Wuppertal 1(DE)**

(72) Erfinder: **Hammann, Ingeborg, Dr.**
**Lutherstrasse 22**
**D-4330 Muelheim/Ruhr(DE)**

## (54) Allenyl-benzylester, Verfahren zu ihrer Herstellung sowie ihre Verwendung zur Schädlingsbekämpfung.

(57) Allenyl-benzylester der Formel I

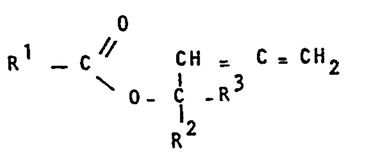

(I)

in welcher
$R^1$ für

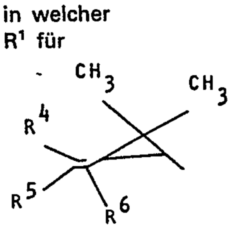

steht, wobei
$R^4$ für Phenyl, das gegebenenfalls durch Halogen, Halogenalkyl, Halogenalkoxy, substituiert ist, steht,
$R^5$ für Wasserstoff oder Halogen steht,
$R^4$ und $R^5$ stehen zusätzlich für Halogen für den Fall, daß $R^3$ für Phenyl steht, das gegebenenfalls durch Halogen oder Benzyl oder Benzyloxy substituiert ist,
$R^6$ für Wasserstoff oder Halogen steht,
sowie für

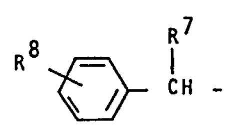

steht, wobei
$R^7$ für Alkyl oder Cycloalkyl steht,
$R^8$ für Wasserstoff sowie einen oder mehrere gleiche oder verschiedene Substituenten der Gruppe Alkyl, Halogen, Halogenalkyl, Alkoxy, Halogenalkoxy, Methylendioxy, Halogenmethylendioxy, Ethylendioxy, Halogenethylendioxy, steht,
$R^2$ steht für Wasserstoff oder niederes Alkyl,
$R^3$ steht für Phenyl, das gegebenenfalls durch Halogen, Phenoxy, Benzyl oder Benzyloxy, wobei letztere drei Substituenten gegebenenfalls durch Halogen substituiert sind, substituiert ist,
oder
für Pyridyl, das gegebenenfalls durch Halogen oder Phenoxy, wobei letzteres gegebenenfalls durch Halogen substituiert ist, substituiert ist, werden erhalten, indem man Alkohole der Formel

./...

EP 0 097 844 A1

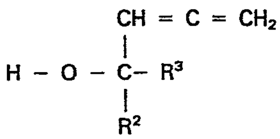

mit Säuren der Formel

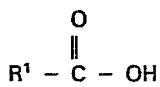

umsetzt. Sie eignen sich als Schädlingsbekämpfungsmittel, insbesondere Insektizide und Akarizide.

BAYER AKTIENGESELLSCHAFT 5090 Leverkusen, Bayerwerk

Zentralbereich
Patente, Marken und Lizenzen Rt/m-c
Ia

# Allenyl-benzylester, Verfahren zu ihrer Herstellung sowie ihre Verwendung zur Schädlingsbekämpfung

Gegenstand der Erfindung sind neue Allenyl-benzylester, ein Verfahren zu ihrer Herstellung sowie ihre Verwendung in Schädlingsbekämpfungsmitteln, insbesondere in Insektiziden und Akariziden.

Es sind inzwischen zahlreiche Benzylester von substituierten Cyclopropancarbonsäuren bekannt geworden (z.B. DE-OS 2 326 077). Diese Verbindungen weisen jedoch neben ihrer guten insektiziden Wirkung den Nachteil ungünstiger Fischtoxizität auf. Verbindungen, die günstige Werte bei der Fischtoxizität besitzen, sind auf der anderen Seite nur mäßig insektizid wirksam. Es bestand daher großes Interesse an Verbindungen mit günstiger Fischtoxizität und guter insektizider Wirkung.

Es sind aus EP-OS 50 093 α-Allenylbenzylester von Dihalogenvinyl-cyclopropancarbonsäuren bekannt. Diese Verbindungen sind jedoch vor allem bei niedrigen Aufwandkonzentrationen nicht immer befriedigend.



BAD ORIGINAL

Es wurden nun neue Allenylbenzylester der Formel I gefunden.

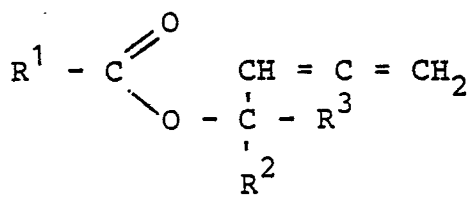

(I)

$R^1$ steht für

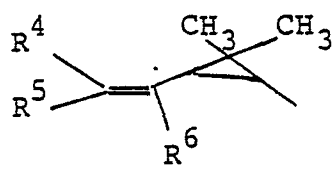

wobei

$R^4$ für Phenyl, das gegebenenfalls durch Halogen, Halogenalkyl, Halogenalkoxy substituiert ist, steht,

$R^5$ für Wasserstoff oder Halogen steht,

$R^6$ für Wasserstoff oder Halogen steht,

$R^4$ und $R^5$ stehen zusätzlich für Halogen für den Fall, daß $R^3$ für Phenyl steht, das gegebenenfalls durch Halogen oder Benzyl oder Benzyloxy substituiert ist,

sowie für

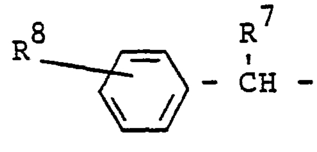

wobei

$R^7$ für Alkyl oder Cycloalkyl steht,

$R^8$ für Wasserstoff sowie einen oder mehrere gleiche oder verschiedene Substituenten aus der Gruppe Alkyl, Halogen, Halogenalkyl, Alkoxy, Halogenalkoxy, Methylendioxy, Halogenmethylendioxy, Ethylendioxy, Halogenmethylendioxy, steht.

$R^2$ steht für Wasserstoff oder niederes Alkyl,

$R^3$ steht für Phenyl, das gegebenenfalls durch Halogen, Phenoxy, Benzyl oder Benzyloxy, wobei letztere drei Substituenten gegebenenfalls durch Halogen substituiert sind, substituiert ist,
oder
für Pyridyl, das gegebenenfalls durch Halogen oder Phenoxy, wobei letzteres gegebenenfalls durch Halogen substituiert ist, substituiert ist.

Die Verbindungen der Formel (I) besitzen, insbesondere unter Berücksichtigung des bei Pyrethroiden verwendbaren Säurerestes, stets mehrere asymmetrische Kohlenstoffatome und können zum Teil im Säurerest auch geometrische Isomerie zeigen. Sie können in Form von Racematen oder verschiedenen optischen Isomeren auftreten. Die Erfindung betrifft sowohl die geometrischen als auch die optischen Isomeren und deren Gemische.

Weiterhin wurde gefunden, daß man die neuen Allenylbenzylester der Formel (I)

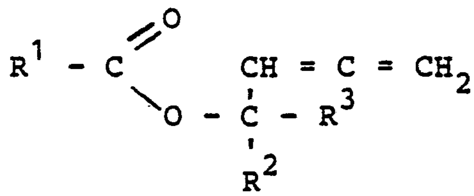

(I)

in welcher

$R^1$ für

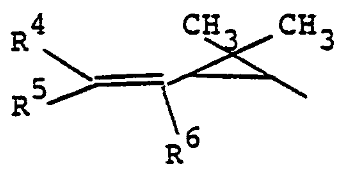

steht, wobei

$R^4$ für Phenyl, das gegebenenfalls durch Halogen, Halogenalkyl, Halogenalkoxy substituiert ist, steht,

$R^5$ für Wasserstoff oder Halogen steht,

$R^6$ für Wasserstoff oder Halogen steht,

$R^4$ und $R^5$ stehen zusätzlich für Halogen für den Fall, daß $R^3$ für Phenyl steht, das gegebenenfalls durch Halogen oder Benzyl oder Benzyloxy substituiert ist,

sowie für

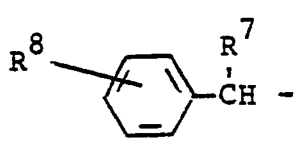

steht, wobei

$R^7$ für Alkyl oder Cycloalkyl steht,

$R^8$ für Wasserstoff sowie einen oder mehrere gleiche oder verschiedene Substituenten aus der Gruppe Alkyl, Halogen, Halogenalkyl, Alkoxy, Halogenalkoxy, Methylendioxy, Halogenmethylendioxy, Ethylendioxy, Halogenmethylendioxy, steht.

$R^2$ steht für Wasserstoff oder niederes Alkyl,

$R^3$ steht für Phenyl, das gegebenenfalls durch Halogen, Phenoxy, Benzyl oder Benzyloxy, wobei letztere drei Substituenten gegebenenfalls durch Halogen substituiert sind, substituiert ist,
oder
für Pyridyl, das gegebenenfalls durch Halogen oder Phenoxy, wobei letzteres gegebenenfalls durch Halogen substituiert ist, substituiert ist,

erhält, indem man Säuren der Formel II

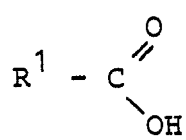

(II)

in welcher
$R^1$ die oben angegebene Bedeutung hat,
oder ihre reaktionsfähigen Derivate,

mit Alkoholen der Formel III

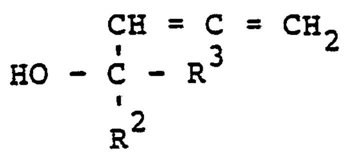

(III)

in welcher
$R^2$ und $R^3$ die oben angegebene Bedeutung haben, oder deren reaktionsfähigen Derivaten, gegebenenfalls in Gegenwart eines Katalysators und gegebenenfalls in Gegenwart eines Säureakzeptors sowie gegebenenfalls in Gegenwart eines Verdünnungsmittels umsetzt.

Die neuen Allenyl-benzylester der Formel (I) zeichnen sich durch ihre insektiziden und akariziden Eigenschaften bei gleichzeitig günstiger Fischtoxizität aus.

Die erfindungsgemäßen Allenyl-benzylester sind durch die Formel (I) allgemein definiert.

In dieser Formel stehen vorzugsweise

$R^1$ für die Gruppierung

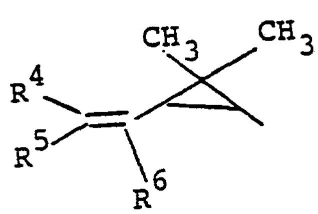

in welcher

$R^4$ für Phenyl steht, das gegebenenfalls durch Fluor, Chlor, Brom, $CF_3$, $OCF_3$, $-CFCl_2$, $-CCl_3$ substituiert ist,

$R^5$ für Fluor, Chlor, Brom, steht,

$R^6$ für Wasserstoff steht.

$R^1$ steht ferner für die Gruppierung

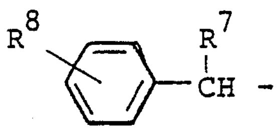

in welcher

$R^8$ für Halogen, insbesondere Fluor, Chlor oder Brom, $C_1$ bis $C_6$-Alkyl, Halogenalkyl mit 1 bis 2 C-Atomen und bis zu 5 Halogenatomen, insbesondere Fluor, Chlor oder Brom, Halogenalkoxy mit 1 bis 2 C-Atomen und bis zu 5 Halogenatomen, insbesondere Fluor, Chlor oder Brom, Methylendioxy oder Ethylendioxy, durch Halogen, insbesondere Fluor, Chlor oder Brom substituiert , steht,

$R^7$ für $C_1$ bis $C_4$-Alkyl oder Cycloalkyl mit bis zu 4 C-Atomen, besonders bevorzugt Isopropyl, steht, und

$R^2$ für Wasserstoff steht und

$R^3$ für mit 0 bis 5 Halogenatomen, insbesondere Fluor, Chlor oder Brom substituiertes Phenyl oder

für gegebenenfalls durch Halogen, insbesondere Fluor, Chlor oder Brom, substituiertes Phenyl, das in 3-Stellung als weiteren Substituenten die gegebenenfalls durch Halogen, insbesondere Fluor, Chlor oder Brom substituierte Phenoxy-, Benzyl- oder Benzyloxygruppe trägt,
oder
für gegebenenfalls mit Fluor, Chlor oder Brom, gegebenenfalls mit Phenoxy substituiertes Pyridyl, wobei die Phenoxygruppe gegebenenfalls durch Fluor, Chlor oder Brom substituiert sein kann.

Bevorzugt sind außerdem die jeweiligen optischen und gegebenenfalls möglichen geometrischen Isomeren.

Als erfindungsgemäße Stoffe der Formel (I) seien beispielsweise genannt

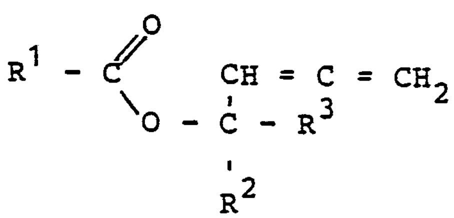

| $R^1$ | $R^2$ | $R^3$ |
|---|---|---|
| 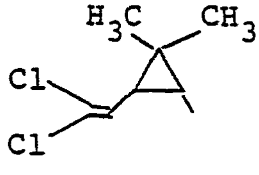 | H | 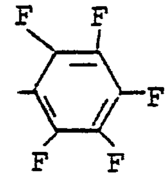 |

| $R^1$ | $R^2$ | $R^3$ |
|---|---|---|
| 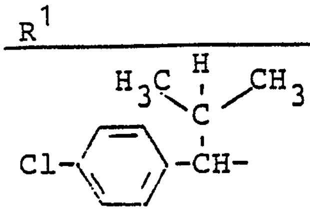 | H | 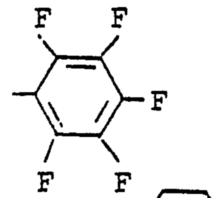 |
| 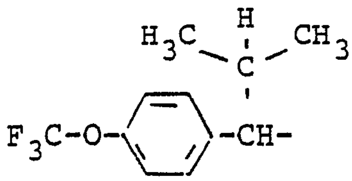 | H | 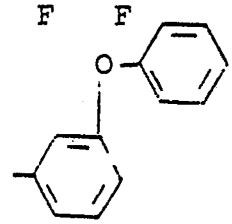 |
| 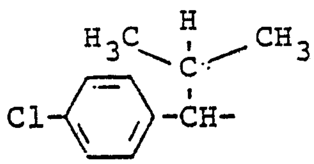 | H | 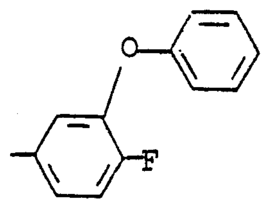 |
| 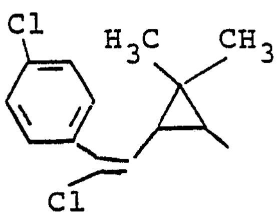 | H | 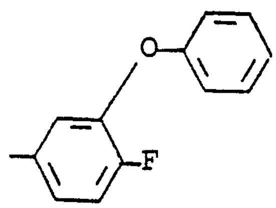 |
| 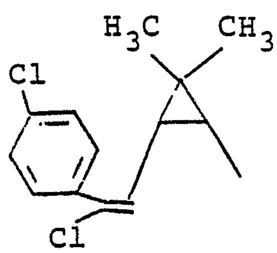 | H | 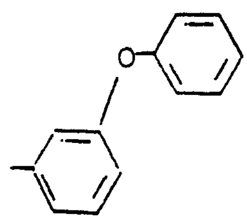 |
| 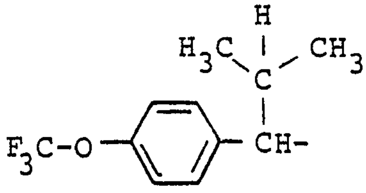 | H | 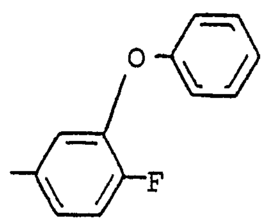 |

Verwendet man beispielsweise 2,2-Dimethyl-3,2'-brom-2'-phenylvinylcyclopropancarbonsäurechlorid und 1-(4'-Fluor-3'-phenoxy-phenyl)-1-hydroxybutadien-(2,3) als Ausgangsstoffe, so kann der Reaktionsverlauf des erfindungsgemäßen Verfahrens durch das nachstehende Formelschema beschrieben werden:

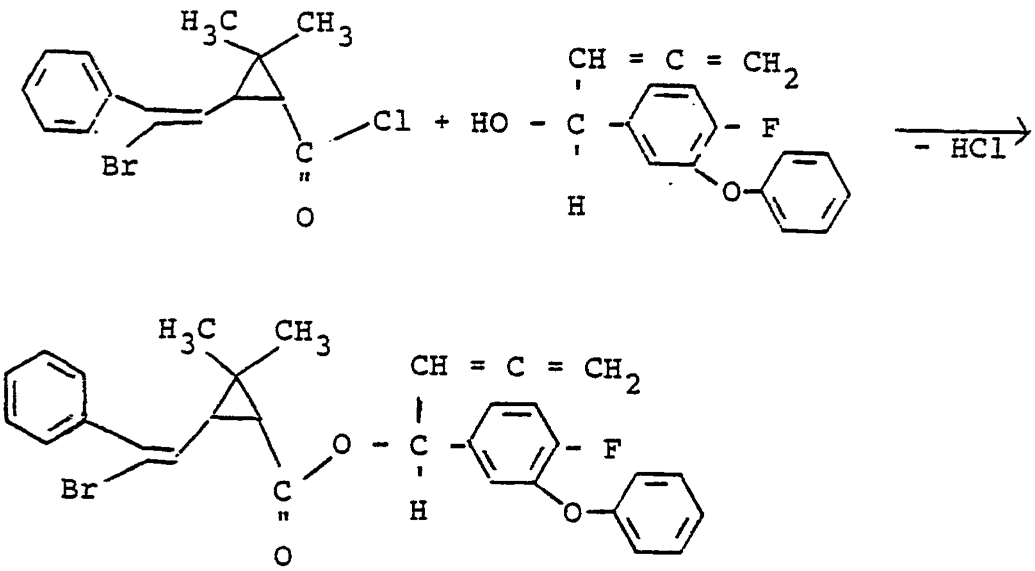


Die bei der Durchführung des erfindungsgemäßen Verfahrens als Ausgangsstoffe verwendbaren Säuren sind bekannt. Sie sind durch die Formel (II) allgemein definiert. In dieser Formel steht $R^1$ vorzugsweise für diejenigen Reste, die bereits im Zusammenhang mit den erfindungsgemäßen Stoffen der Formel (I) die vorzugsweise für diesen Rest genannten Bedeutungen besitzen.

Als reaktionsfähige Derivate der Säuren der Formel (II) sind vorzugsweise deren Säurehalogenide, insbesondere Chloride oder Anhydride zu verstehen.

Die Säuren der Formel (II) sowie deren reaktionsfähige Derivate sind bekannt (vgl. R. Wegler, s.o., Bd. 7, Seite 91 ff sowie die dort zitierte Originalliteratur).

Die bei der Durchführung des erfindungsgemäßen Verfahrens ebenfalls als Ausgangsstoffe verwendbaren Alkohole sind durch die Formel (III) allgemein definiert. In dieser Formel stehen $R^2$ und $R^3$ vorzugsweise ebenfalls für diejenigen Reste, die bereits im Zusammenhang mit den erfindungsgemäßen Stoffen der Formel (I) vorzugsweise für diese Reste genannt wurden.

Als reaktionsfähige Derivate der Alkohole der Formel (III) sind vorzugsweise Ester, insbesondere Methansulfonyl-, Benzolsulfonyl- oder Toluolsulfonylester, die nach allgemein üblichen Verfahren aus diesen Alkoholen erhältlich sind, zu verstehen.

Als Katalysator für das erfindungsgemäße Verfahren kommen alle für Veresterungsreaktionen dieses Typs in der organischen Chemie üblichen Katalysatoren in Betracht. Es seien beispielsweise genannt als wasserbindende Mittel Schwefelsäure oder Carbodiimide, insbesondere Dicyclohexylcarbodiimid, als saure Katalysatoren ebenfalls Schwefelsäure, Sulfonsäuren wie Methan-, Benzol- oder Toluolsulfonsäure, Halogenwasserstoffverbindungen oder Phosphorsäure, als basische Katalysatoren Amine wie beispielsweise 4-Dimethylaminopyridin.

Als Säureakzeptor können ebenfalls alle üblichen Säurebindemittel verwendet werden, beispielsweise anorganische Basen, wie Alkali- oder Erdalkalihydroxide, anorganische Salze wie Soda, Pottasche oder Kalk, aber auch organische Basen, vorzugsweise Amine, wie beispielsweise Triethylamin, Pyridin, Chinolin, Diazabicyclooctan, Diazabicyclononen oder Diazabicycloundecen.

Als Verdünnungsmittel können bei der Durchführung des erfindungsgemäßen Verfahrens praktisch alle inerten organischen Solventien verwendet werden. Vorzugsweise in Betracht kommen aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Dichlormethan, Chloroform, Chlorethan, Tetrachlorkohlenstoff, Chlorbenzol und Dichlorbenzol, ferner Ether, wie Diethylether, Dibutylether, Glycoldimethylether, Diglycoldimethylether, Tetrahydrofuran und Dioxan, sowie Ketone, wie Aceton, Butanon, Methylisopropylketon oder Methylisobutylketon, außerdem Ester, wie Essigsäuremethyl- oder -ethylester, oder Nitrile, wie beispielsweise Acetonitril oder Propionitril, weiterhin Amide, wie z.B. Dimethylformamid, Dimethylacetamid und N-Methylpyrrolidon, sowie polare Solventien wie Dimethylsulfoxid, Tetramethylensulfon oder Hexamethylphosphorsäuretriamid.

Die Reaktionstemperaturen können bei der Durchführung des erfindungsgemäßen Verfahrens innerhalb eines großen Bereiches variiert werden. Man arbeitet im allgemeinen bei Temperaturen zwischen -40°C und +150°C, vorzugsweise zwischen 0°C und +120°C.

Das erfindungsgemäße Verfahren wird im allgemeinen bei Normaldruck durchgeführt.

Die Ausgangsstoffe der Formeln (II) und(III) werden in der Regel in etwa äquimolaren Mengen eingesetzt. Ein Überschuß der einen oder anderen Komponente ist möglich. Die Umsetzung wird im allgemeinen in einem geeigneten Verdünnungsmittel, dessen Menge sich vorzugsweise nach der Rührbarkeit und leichten Temperaturkontrolle bei der Reaktion richtet, und bevorzugt in Gegenwart eines Katalysators oder eines Säureakzeptors durchgeführt. Die Katalysatormenge kann von 0,001 % bis zu etwa äquimolarem Verhältnis variiert werden, der Säureakzeptor kann bis zum mehrfachen Überschuß, vorzugsweise in äquimolarer bis doppelter Menge, dosiert werden. Das Reaktionsgemisch wird im Regelfall mehrere Stunden gerührt. Die Aufarbeitung des Reaktionsumsatzes erfolgt nach üblichen Methoden (siehe hierzu auch die experimentellen Beispiele).

Die neuen Verbindungen fallen zum Teil in Form von Ölen an, die meist nicht unzersetzt destilliert werden können. Sie lassen sich jedoch durch längeres vorsichtiges Aufheizen im Vakuum auf mäßig erhöhte Temperaturen von leichter flüchtigen Anteilen befreien und auf diese Weise reinigen. Ebenfalls zur Reinigung herangezogen werden können die allgemein bekannten Methoden der Chromatographie, beispielsweise der Flüssigkeitschromatographie an stationärer Phase wie Kieselgel oder Aluminiumoxid mit Lösungsmittelgemischen wie Cyclohexan/Aceton; Cyclohexan/Essigester oder vergleichbaren Laufmitteln oder deren Gemischen.

Zur Charakterisierung der neuen Substanzen dient der Brechungsindex. Soweit die neuen Verbindungen in fester Form anfallen, können sie durch Umkristallisation gereinigt werden. In diesem Fall dient der Schmelzpunkt zur Charakterisierung.

Die bei der Durchführung des erfindungsgemäßen Verfahrens als Ausgangsstoffe verwendbaren Alkohole der Formel (III) sind zum Teil bekannt (EP-OS 50 093).

Sie lassen sich nach den dort beschriebenen Verfahren herstellen. Beispielsweise wird 1-(3-Benzyl-phenyl)-propin-(2)-ol-(1) mit Formaldehyd, Diisopropylamin und Kupfer-(I)-bromid als Katalysator umgesetzt. Für diesen Fall läßt sich die Umsetzung durch das folgende Reaktionsschema beschreiben:

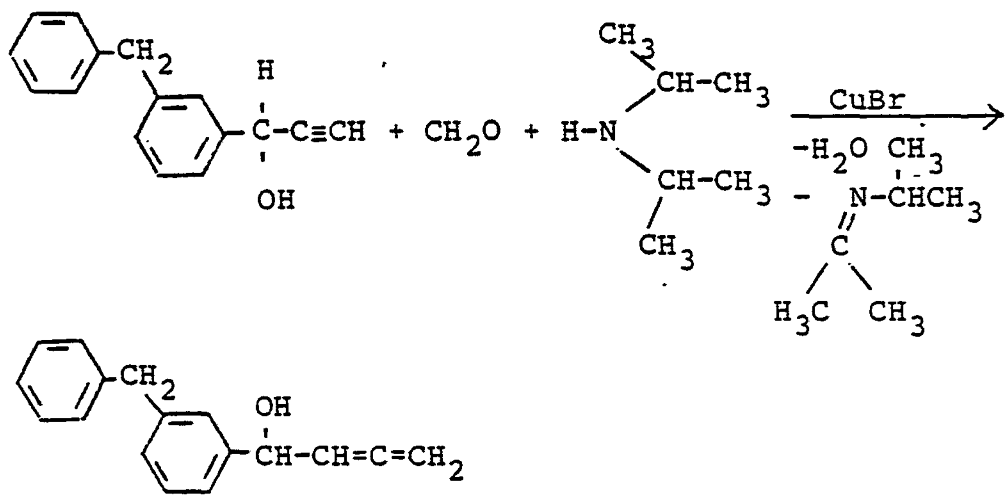


Die bei der Durchführung dieser Reaktion als Ausgangsstoff verwendbaren Alkinverbindungen sind bekannt.

0097844

Die Wirkstoffe eignen sich bei guter Pflanzenverträglichkeit und günstiger Warmblütertoxizität zur Bekämpfung von tierischen Schädlingen, insbesondere Insekten oder Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen,

durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele:

1. 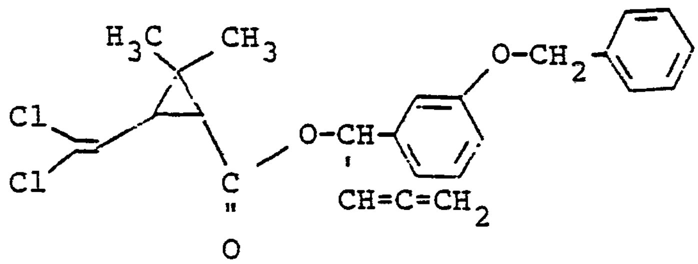

2,5 g (0,01 Mol) 1-Hydroxy-1-m-benzyloxyphenyl-butadien-(2,3), 20 ml Toluol, 20 ml Petrolether und 1 g (0,013 Mol) Pyridin werden bei Raumtemperatur und Feuchtigkeitsausschluß mit 2,3 g (0,01 Mol) 2,2-Dimethyl-3-2',2'-dichlorvinyl-cyclopropancarbonsäurechlorid versetzt und bei gleicher Temperatur über Nacht stehen gelassen. Nach Aufnehmen in 200 ml $CH_2Cl_2$, zweimaligem Waschen mit Wasser, Trocknen über Natriumsulfat, Einengen und Abziehen der flüchtigen Anteile im Vakuum bis 100 °C/0,3 wird der Rückstand auf 20 g Kieselgel aufgezogen und mit Cyclohexan/Aceton ansteigender Polarität das Produkt eluiert, wobei sich in der Hauptfraktion 2 g eines hellen Öls (47 %) ergeben, $n_D^{20}$ 1.5800.

2. 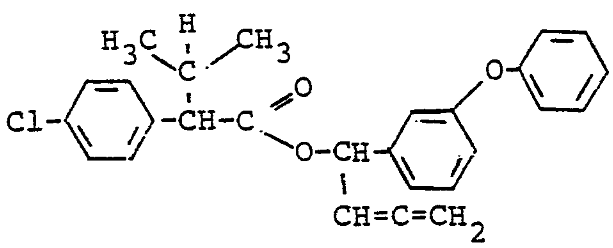 $n_D^{20}$ 1.5900

3. 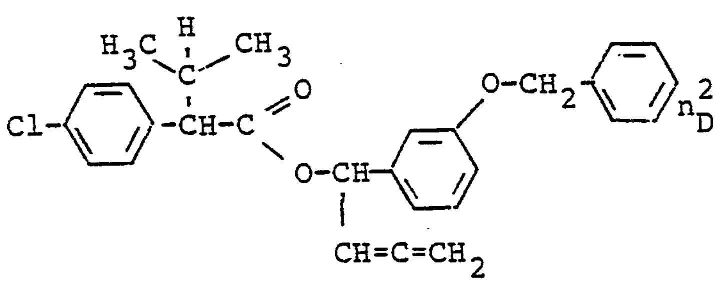 $n_D^{20}$ 1.5970

4) 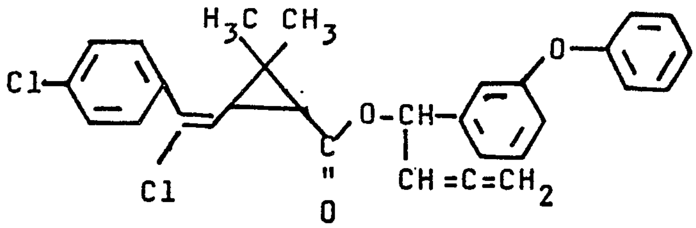

trans-Z

$n_D^{20}$ = 1,6050

5) 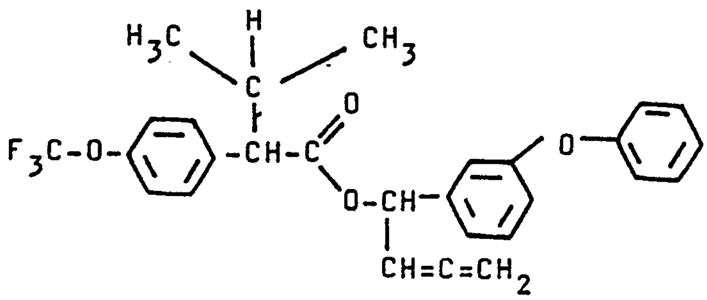

$n_D^{20}$ = 1,539

Vorprodukte:

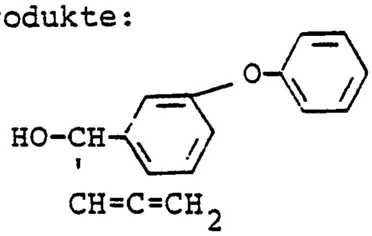

$C_{16}H_{14}O_2$

22 g (0,1 Mol) 1-m-Phenoxyphenyl-propargylalkohol, 4,4 g (0,15 Mol) Paraformaldehyd, 4 g (0,028 Mol) Kupfer-I-bromid, 11 g (0,11 Mol) Diisopropylamin und 100 ml absolutes Dioxan werden unter Feuchtigkeitsausschluß 3 Stunden unter Rückfluß erhitzt. Nach beendeter Reaktion wird der Ansatz in 100 ml Dichlormethan aufgenommen, filtriert, mit verdünnter Salzsäure gewaschen, über $Na_2SO_4$ getrocknet und eingeengt im Vakuum. Das verbliebene dunkle Öl wird nach Aufziehen auf 30 g Kieselgel mit Cyclohexan/Aceton steigender Polarität wieder eluiert, wobei sich in der Hauptfraktion 8 g eines hellen Öls (35 %) ergeben, $n_D^{20}$ 1.6071.

In gleicher Weise kann erhalten werden

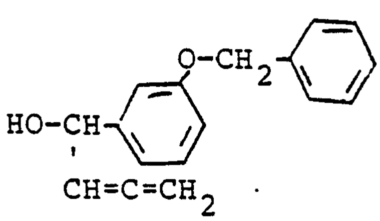

$C_{17}H_{16}O_2$

$n_D^{20}$ 1.5889

Beispiel A

Tetranychus-Test (resistent)

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 2

Beispiel B

Laphygma-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2

Patentansprüche:

1. Allenyl-benzylester der Formel I

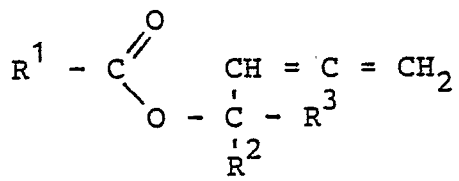

(I)

in welcher

$R^1$ für

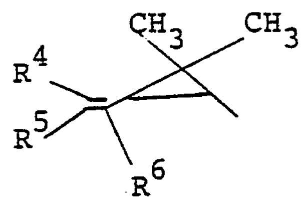

steht, wobei

$R^4$ für Phenyl, das gegebenenfalls durch Halogen, Halogenalkyl, Halogenalkoxy, substituiert ist, steht,

$R^5$ für Wasserstoff oder Halogen steht,

$R^4$ und $R^5$ stehen zusätzlich für Halogen für den Fall, daß $R^3$ für Phenyl steht, das gegebenenfalls durch Halogen oder Benzyl oder Benzyloxy substituiert ist,

$R^6$ für Wasserstoff oder Halogen steht,

sowie für

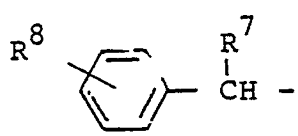

steht, wobei

$R^7$ für Alkyl oder Cycloalkyl steht,

$R^8$ für Wasserstoff sowie einen oder mehrere gleiche oder verschiedene Substituenten der Gruppe Alkyl, Halogen, Halogenalkyl, Alkoxy, Halogenalkoxy, Methylendioxy, Halogenmethylendioxy, Ethylendioxy, Halogenethylendioxy, steht.

$R^2$ steht für Wasserstoff oder niederes Alkyl,

$R^3$ steht für Phenyl, das gegebenenfalls durch Halogen, Phenoxy, Benzyl oder Benzyloxy, wobei letztere drei Substituenten gegebenenfalls durch Halogen substituiert sind, substituiert ist,
oder
für Pyridyl, das gegebenenfalls durch Halogen oder Phenoxy, wobei letzteres gegebenenfalls durch Halogen substituiert ist, substituiert ist.

2. Verfahren zur Herstellung der Allenyl-benzylester der Formel (I)

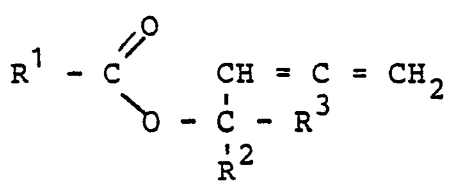

(I)

in welcher

$R^1$ für

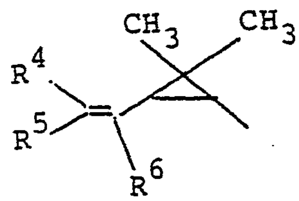

steht, wobei

$R^4$ für Phenyl, das gegebenenfalls durch Halogen, Halogenalkyl, Halogenalkoxy, substituiert ist, steht,

$R^5$ für Wasserstoff oder Halogen steht,

$R^4$ und $R^5$ stehen zusätzlich für Halogen für den Fall, daß $R^3$ für Phenyl steht, das gegebenenfalls durch Halogen oder Benzyl oder Benzyloxy substituiert ist,

$R^6$ für Wasserstoff oder Halogen steht,

sowie für

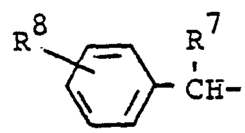

steht, wobei

$R^7$ für Alkyl oder Cycloalkyl steht,

$R^8$ für Wasserstoff sowie einen oder mehrere gleiche oder verschiedene Substituenten der Gruppe Alkyl, Halogen, Halogenalkyl, Alkoxy, Halogenalkoxy, Methylendioxy, Halogenmethylendioxy, Ethylendioxy, Halogenethylendioxy, steht.

$R^2$ steht für Wasserstoff oder niederes Alkyl,

$R^3$ steht für Phenyl, das gegebenenfalls durch Halogen, Phenoxy, Benzyl oder Benzyloxy, wobei letztere drei Substituenten gegebenenfalls durch Halogen substituiert sind, substituiert ist,
oder
für Pyridyl, das gegebenenfalls durch Halogen oder Phenoxy, wobei letzteres gegebenenfalls durch Halogen substituiert ist, substituiert ist,

indem man Säuren der Formel II

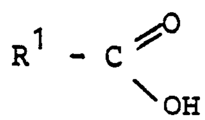

(II)

in welcher
$R^1$ die oben angegebene Bedeutung hat,
oder ihre reaktionsfähigen Derivate,
mit Alkoholen der Formel III

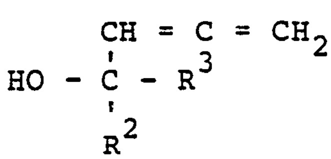

(III)

in welcher
$R^2$ und $R^3$ die oben angegebene Bedeutung haben,
oder ihre reaktionsfähigen Derivate,

gegebenenfalls in Gegenwart eines Katalysators und gegebenenfalls in Gegenwart eines Säureakzeptors sowie gegebenenfalls in Gegenwart eines Verdünnungsmittels,
umsetzt.

3. Schädlingsbekämpfungsmittel, gekennzeichnet durch einen Gehalt an mindestens einem Allenyl-benzylester der Formel (I).

4. Verwendung von Allenyl-benzylester der Formel (I) zur Bekämpfung von Schädlingen.

5. Verfahren zur Bekämpfung von Schädlingen, dadurch gekennzeichnet, daß man Allenyl-benzylester der Formel (I) auf Schädlinge und/oder ihren Lebensraum einwirken läßt.

6. Verfahren zur Herstellung von Schädlingsbekämpfungsmitteln, dadurch gekennzeichnet, daß man Allenylbenzylester der Formel (I) mit Streckmitteln und/oder oberflächenaktiven Mitteln vermischt.

Europäisches Patentamt

# EUROPÄISCHER RECHERCHENBERICHT

0097844
Nummer der Anmeldung

EP 83 10 5538

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A,D | EP-A-0 050 093 (CIBA-GEIGY) * Ansprüche * --- | 1-6 | C 07 C 69/612<br>C 07 C 69/734<br>C 07 C 69/743<br>A 01 N 31/14<br>A 01 N 53/00 |
| P,X | DE-A-3 229 661 (CIBA-GEIGY) * Ansprüche * ----- | 1-6 | |
| | | | **RECHERCHIERTE SACHGEBIETE (Int. Cl. ³)** |
| | | | C 07 C 69/00 |

Der vorliegende Recherchenbericht wurde für alle Patentansprüche erstellt.

| Recherchenort | Abschlußdatum der Recherche | Prüfer |
|---|---|---|
| DEN HAAG | 16-09-1983 | WRIGHT M.W. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument